# EUROPEAN PATENT SPECIFICATION

(11) **EP 0 312 368 B1**
(45) Date of publication and mention of the grant of the patent: **29.12.1993**
(21) Application number: 88309619.0
(22) Date of filing: 13.10.1988
(51) Int. Cl.: A61F 5/56

(54) **A device for preventing snoring**
Vorrichtung zum Verhindern des Schnarchens
Dispositif pour empêcher le ronflement

(30) Priority: 13.10.1987 US 107670
(43) Date of publication of application: 19.04.1989
(73) Proprietor: HAYS & MEADE, INC., Albuquerque New Mexico 87107 (US)
(72) Inventor: Hays, Marvin B., Albuquerque New Mexico 87123 (US); Meade, Thomas E., Albuquerque New Mexico 87107 (US)
(74) Representative: UEXKÜLL & STOLBERG

(56) References cited:
- DE-A- 2 320 501
- GB-A- 1 569 129
- US-A- 1 674 336
- US-A- 3 132 647
- US-A- 3 434 470
- US-A- 4 304 227
- US-A- 4 669 459

## Description

The present invention relates to a device for preventing snoring and more particularly to such a device which is intended to be worn in the mouth of a user.

Various snore reducing structures are known in the art, as well as other oral devices to be received in the mouth to prevent or reduce damage to the oral cavity during athletic competition, such as boxing.

Athletic mouth guards are intended to prevent the abrading of the soft tissue of the lips and cheeks against the teeth and to prevent the upper and lower teeth from forceably engaging against one another, with resultant and permanent damage.

A typical prior art mouth guard consists of an integrally formed plastics member having a front wall which extends over the front surfaces of the upper and lower teeth and an inwardly projecting portion which is received between the upper and lower teeth when the guard is worn.

Snoring is a sound which generally emerges from the mouth of a person who is asleep. It has been observed that snoring is closely associated with breathing through an open mouth. However, it is believed that vibration of the uvula and soft palate contribute to the sound of snoring.

Various wearable snore reducing devices are known in the prior art. Some are arranged to seal the lips of the wearer, one to another, thus blocking the mouth, and preventing breathing through the mouth. Others recognize that portions of the uvula and soft palate vibrate during sleep in response to the passage of air past these tissues and therefore attempt to minimize such vibrations by sharply reducing the volume of air passing through the mouth without necessarily completely blocking the mouth. If the nose is blocked, or partially blocked, this reduction of the airway increases the velocity of the air passing those tissues and snoring can actually increase.

US-A-1 674 336 discloses a device for preventing snoring, which comprises a generally U-shaped member to be received in the mouth of a user. The device keeps the upper and lower jaw in their normal positions without shifting them in any direction.

None of the prior art proposals provide an entirely satisfactory solution to the problem of snoring.

The present invention seeks to provide a device which may be inserted into the mouth of a user in order to eliminate or reduce snoring during sleep.

According to one aspect of the present invention there is provided a device for preventing snoring, said device being provided for removable placement in the mouth of a patient to prevent or reduce snoring wherein the device maintains a gap between and relative orientation of the upper and lower teeth of that patient to thereby prevent or reduce snoring experienced by that patient, the device comprising:
a U-shaped member having a curved shape which substantially corresponds to the shape of the patient's upper dental arch, said U-shaped member having an upwardly open tooth receiving trench for receiving and engaging at least a portion of at least some of the upper teeth of the patient; and
an aperture for allowing the passage of air into and from the mouth; characterized by:
a ramp means extending rearwardly from a lower edge of said U-shaped member, said ramp means engaging at least some of the lower anterior teeth of the patient, said U-shaped member and the ramp means being configured for a particular patient so that when the upper teeth of that patient are received and engaged by the tooth receiving trench of the U-shaped member, normal jaw motion will cause some of the lower teeth to engage against the underside of the ramp, which will cam the lower jaw forward whereby the lower teeth lie in front of the upper teeth and there is a space in between the upper and lower teeth and between the lips, whereby, compared to the normal position, an increased spacing is created between the tongue and the palate and uvula as well as between the tongue and the rerar wall of the pharynx is also increased, which reduces the velocity of air passing along the air-way via said aperture during breathing, and palate and uvula vibrations are reduced or eliminated because the soft tissues previously entrained by the passage of air no longer vibrate or oscillate or vibrate at a reduced amplitude.

Thus, in contradistinction to the teachings discussed above, the present invention opens the mouth's posterior airway in response to natural mouth movements and motions of the jaws. This opening of the airway serves to reduce the velocity of the air surrounding and moving past the uvula and soft palate whereby the tissues thus entrained no longer vibrate, or the amplitude of vibration is sharply reduced.

The ramp structure of the device enables natural jaw motions to cam the lower jaw into a forward position thereby elevating the hyoid bone forming a more open posterior airway. The hyoid bone supports the tongue and gives attachment to its numerous muscles.

Preferably the generally U-shaped member has an upper rim corresponding in shape to the user's upper dental arch and the means for removably mounting the device upon the upper set of teeth comprise a trench or channel adapted to receive and engage at least some of the user's upper teeth.

Conveniently the trench or channel is defined between an arcuate front wall and an arcuate rear wall and the ramp means are located below the arcuate walls and extend rearwardly from the central part of said front wall.

Advantageously the ramp means forms an angle of approximately 60° when viewed in side elevation.

Preferably the U-shaped member defines a space or cavity between said rear wall and an upper surface of the ramp means. This space or cavity serves to guide the tongue of the user into a position forward of its usual resting position behind the lower teeth, whereby the normal spacing between the structure of the tongue and the uvula and rear wall of the pharynx is increased.

Conveniently the aperture in the U-shaped member is formed at the mid-point of the front wall of the member, the aperture being of generally rectangular configuration.

Advantageously the trench of channel formed in the U-shaped member is configured to extend from the premolar teeth on one side of the mouth of a user forwardly in congruence with the user's dental arch to terminate at the premolar teeth on the other side of the user's mouth.

The device may be moulded of a synthetic plastics material of the acrylic group, such as methyl-methacrylate.

In order that the invention may be more readily understood and so that further features thereof may be appreciated the invention will now be described by way of example with reference to the accompanying drawings in which:
FIGURE 1 is a perspective view of an oral device of the invention;
FIGURE 2 is a rear view of the oral device showing a trench structure designed to receive the upper dental arch of the wearer;
FIGURE 3 is a side elevation view of the oral device;
FIGURE 4 is a front view of the oral device looking in the direction of the arrow IV appearing in Figure 1;
FIGURE 5 is a partial side elevation in cross section of a human head and neck; and
FIGURE 6 is a partial side elevation in cross section of the human head and neck of Figure 5 showing the oral device in place.

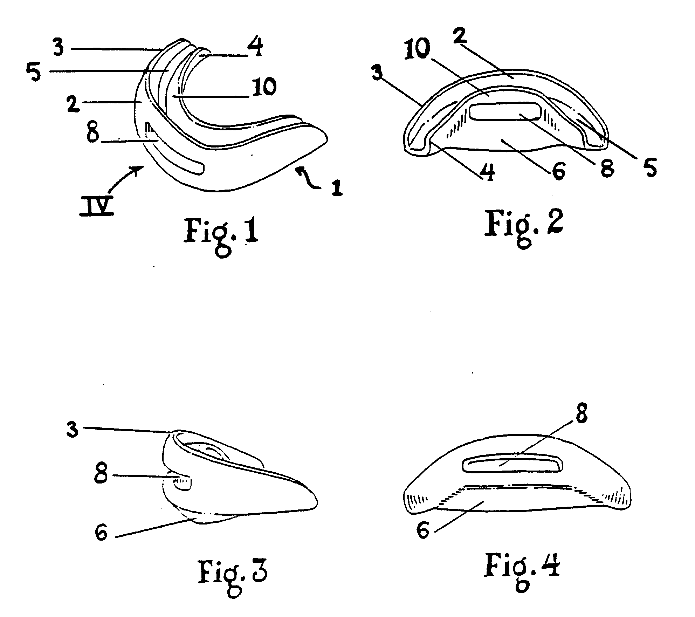

As illustrated in Figures 1-4, inclusive, an oral device for preventing snoring comprises a structure shaped to conform to the upper dental arch of a user and extending at least between the pre-molar teeth on each side of the wearer's mouth. The device is integrally formed from a single piece of methylmethacrylate which is a plastic material commonly used for dentures.

Referring to Figures 1-4 of the drawings, the device comprises a generally semi-circular or U-shaped member 1. The U-shaped member 1 has an arcuate front wall 2 having an upper rim 3 which corresponds in shape to the user's upper dental arch i.e., the shape of the user's upper teeth. An arcuate rear wall 4 is spaced behind the front wall 2 and a generally U-sectioned, teeth receiving trench or channel 5 is defined between the front wall 2 and the rear wall 4. In side elevation, as can be seen in Figure 3, the U-shaped member tapers towards the rear of the device i.e., the height of the front wall 2 decreases as the wall extends around the sides of the device towards the rear thereof.

At the bottom of the device, at the front, a curved ramp 6 extends rearwardly from the central region of the lower edge of the front wall 2. The ramp 6 extends rearwardly at an angle of approximately 60° and extends to a position located laterally between the lower edges of the rear wall 4, as is seen most clearly in Figure 2 of the drawings.

An aperture 8 extends through the front wall 2 and the rear wall 4 to provide an opening extending through the device at a position located centrally of the device i.e., an opening through the base of the U-shaped member. The aperture 8 is of generally rectangular configuration and is located above the forwardmost region of the ramp 6 and beneath the trench or channel 5. The aperture extends through a central region 10 of the rear wall 4. The central region 10 of the rear wall 4 and the upper surface of the ramp 6 serve to define a space or cavity which is effectively within the U-shaped member.

The device is adapted to be placed within the mouth upon the dental arch defined by the upper teeth. The device is intended to be formed specifically for a particular wearer and to be fitted to the wearer by a professional in the dental arts such that when received in the mouth it will grip some, at least, of the upper teeth, and is thus retained in that oral cavity. The trench or channel 5 is therefore configured to correspond to the contours of the wearers upper teeth so that when the device is worn the trench engages and grips the teeth to retain the device in position whilst at the same time the device may be easily removed from the teeth without difficulty. The trench therefore constitutes means for removeably mounting the device upon the teeth. After fitting by a professional, the device is cured to prevent absorption of mouth fluids or cleaning fluids and to present a smooth non-irritating surface to the soft tissues of the mouth.

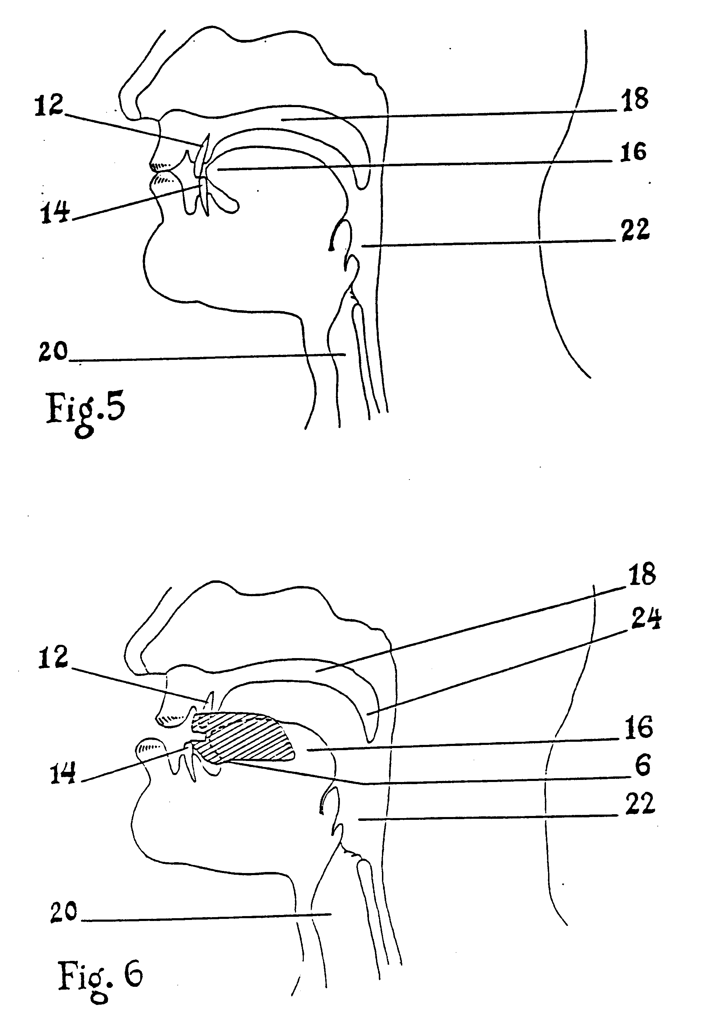

Referring now to Figures 5 and 6, Figure 5 shows the normal position of the teeth, jaw and air passages wherein upper teeth 12 overlie and are in front of lower teeth 14. The tip of a tongue 16 lies against the lower teeth 14 as shown. The palate 18 terminates at the back of the mouth in the uvula 24 and a normal spacing is shown between these members and the tongue 16. Air passages lead to the trachea 20, which lies next to the esophagus 22.

When the device of this invention is in situ as revealed in Figure 6, the upper teeth 12 are received in the trench formed between the front wall 2 and the rear wall 4 (Figures 1 and 2). In order for the device to be worn comfortably and to be easily removable and yet to be firmly retained upon the teeth 12 during sleep, it must be individually fitted to the user by a professional dentist. Normal mouth motions such as clenching of the jaw will cause some of the lower teeth to engage against the underside of the ramp 6, and the result of such engagement is to cam the lower jaw forward whereby the lower teeth 14 lie in front of the upper teeth 12 and there is a spacing between the upper and lower teeth and between the lips. This engagement also causes the hyoid bone supporting the tongue to be elevated. The result of this interaction on airway structures is disclosed in Figure 6 where, as compared with Figure 5, an increased spacing is seen between the tongue and the palate and uvula. The spacing between the tongue and the rear wall of the pharynx is also increased. This increase in the volume of the airway reduces the velocity of air passing along the airway during breathing, and palate and uvula vibrations are reduced or eliminated because the soft tissues previously entrained by the passage of air no longer vibrate or oscillate or vibrate at a reduced amplitude.

The aperture 8 serves a dual function in the structure of the device. If the nose is blocked or partially blocked, it allows for the passage of air for breathing through the mouth. If the nose is unblocked, the tongue, which by the nature of its nerve responses seeks a cavity, as anyone who has a cavity in a tooth will bear witness, will seek the space or cavity formed between the upper surface of the ramp 6 and the central region 10 of the rear wall 4 (Figure 2) and will engage the aperture 8 thus elongating itself and further increasing its distance from the palate and uvula and further increasing the volume of the airway.

The features disclosed in the foregoing description, in the following claims and/or in the accompanying drawings may, both separately and in any combination thereof, be material for realising the invention in diverse forms thereof.

## Claims

1. A device for removable placement in the mouth of a patient to prevent or reduce snoring wherein the device maintains a gap between and relative orientation of the upper and lower teeth of that patient to thereby prevent or reduce snoring experienced by that patient, the device comprising:
a U-shaped member (91) having a curved shape which substantially corresponds to the shape of the patient's upper dental arch, said U-shaped member having an upwardly open tooth receiving trench (5) for receiving and engaging at least a portion of at least some of the upper teeth (12) of the patient; and
an aperture (8) for allowing the passage of air into and from the mouth; characterized by:
a ramp means (6) extending rearwardly from a lower edge of said U-shaped member, said ramp means (6) engaging at least some of the lower anterior teeth (14) of the patient, said U-shaped member and the ramp means being configured for a particular patient so that when the upper teeth (12) of that patient are received and engaged by the tooth receiving trench (5) of the U-shaped member, normal jaw motion will cause some of the lower teeth to engage against the underside of the ramp (6), which will cam the lower jaw forward whereby the lower teeth (14) lie in front of the upper teeth (12) and there is a space in between the upper and lower teeth and between the lips, whereby, compared to the normal position, an increased spacing is created between the tongue and the palate and uvula as well as between the tongue and the rerar wall of the pharynx is also increased, which reduces the velocity of air passing along the air-way via said aperture (8) during breathing, and palate and uvula vibrations are reduced or eliminated because the soft tissues previously entrained by the passage of air no longer vibrate or oscillate or vibrate at a reduced amplitude.

2. A device as in claim 1, wherein the aperture (8) extends through the device at a position located centrally of the device.

3. A device according to Claim 1 wherein the ramp means (6) forms an angle of approximately 60° when viewed in side elevation.

4. A device according to any one of the preceding claims wherein the device is moulded of a synthetic plastics material of the acrylic group.

5. A device according to Claim 4 wherein the device is moulded of methyl-methacrylate.

## Patentansprüche

1. Vorrichtung für ein herausnehmbares Plazieren im Mund eines Patienten, um ein Schnarchen zu verhindern oder zu reduzieren, wobei die Vorrichtung einen Spalt zwischen den Zähnen des Ober- und des Unterkiefers und die relative Stellung derselben beim Patienten aufrechterhält, um dadurch ein Schnarchen zu verhüten oder zu reduzieren, das der Patient erfährt, wobei die Vorrichtung umfaßt:
ein U-förmiges Element (1), das eine gekrümmte Gestalt hat, welche im wesentlichen der Form des oberen Zahnbogens des Patienten entspricht, wobei dieses U-förmige Element eine nach oben offene die Zähne aufnehmende Rinne (5) hat, um mindestens einen Teil zumindest einiger Zähne des Oberkiefers (12) des Patienten aufzunehmen und mit diesen in Eingriff zu kommen; und
eine Öffnung (8), um das Durchtreten von Luft in den Mund und aus diesem heraus zu gestatten; **gekennzeichnet durch:**
ein Rampenmittel (6), das sich nach hinten von einer Unterkante des U-förmigen Elements aus erstreckt, wobei dieses Rampenmittel (6) mit mindestens einem Teil der vorderen Zähne des Unterkiefers (14) des Patienten in Eingriff kommt, wobei dieses U-förmige Element und das Rampenmittel so gestaltet sind, daß dann, wenn die Zähne des Oberkiefers (12) des Patienten durch die die Zähne aufnehmende Rinne (5) des U-förmigen Elements aufgenommen werden und mit diesen in Eingriff kommen, eine normale Kieferbewegung verursacht, daß einige Zähne des Unterkiefers gegen die Unterseite der Rampe (6) zum Eingriff kommen, was den Unterkiefer kurvenförmig nach vorn schiebt, wodurch die Zähne des Unterkiefers (14) vor den Zähnen des Oberkiefers (12) liegen und es einen Raum zwischen den Zähnen des Ober- und des Unterkiefers und zwischen den Lippen gibt, wodurch verglichen mit der Normalstellung ein erhöhter Abstand zwischen der Zunge und dem weichen Gaumen und dem Gaumenzäpfchen geschaffen und auch der zwischen der Zunge und der Rückwand des Rachens vergrößert wird, was die Geschwindigkeit von Luft herabsetzt, die entlang des Luftweges über diese Öffnung (8) während des Atmens gelangt und die Schwingungen von weichem Gaumen und Gaumenzäpfchen reduziert oder beseitigt werden, weil das weiche Gewebe, das vorher durch das Durchtreten von Luft mitgerissen worden ist, nicht mehr schwingt oder mit einer reduzierten Amplitude schwingt.

2. Vorrichtung nach Anspruch 1, wobei sich die Öffnung (8) durch die Vorrichtung in einer Position erstreckt, die in der Mitte der Vorrichtung liegt.

3. Vorrichtung nach Anspruch 1, wobei das Rampenmittel (6) einen Winkel von ungefähr 60° bildet, wenn man es im Seitenaufriß sieht.

4. Vorrichtung nach einem der vorhergehenden Ansprüche, wobei die Vorrichtung aus einem synthetischen Plastmaterial der Akrylgruppe formgegossen ist.

5. Vorrichtung nach Anspruch 4, wobei die Vorrichtung aus Methylmetakrylat formgegossen ist.

## Revendications

1. Dispositif à placer amoviblement dans la bouche d'une personne ou d'un patient pour éviter ou diminuer le ronflement, ce dispositif maintenant un intervalle entre les dents supérieures et les dents inférieures de cette personne et maintenant une orientation relative des dents supérieures et des dents inférieures de la personne pour éviter ainsi ou diminuer le ronflement affectant cette personne, le dispositif comprenant :
un élément (1) en forme de U ayant une forme incurvée qui correspond sensiblement à la forme de l'arcade dentaire supérieure de la personne, ledit élément en forme de U ayant une tranchée (5) ouverte sur le dessus pour loger et recevoir les dents, pour recevoir au moins une partie d'au moins certaines des dents supérieures (12) de la personne et venir au contact de ces dents ; et
une ouverture (8) pour laisser l'air passer pour pénétrer dans la bouche et sortir de cette bouche, dispositif caractérisé par :
une rampe (6) qui s'étend vers l'arrière, depuis un bord inférieur dudit élément en forme de U, cette rampe (6) venant au contact d'au moins certaines des dents (14) antérieures inférieures de la personne, ledit élément en forme de U et la rampe étant configurés pour une personne particulière de sorte que, lorsque les dents supérieures (12) de cette personne sont reçues et logées dans la tranchée (5) de réception des dents de l'élément en forme de U et viennent au contact de cet élément, un mouvement normal de la mâchoire va provoquer un contact de certaines des dents inférieures contre le côté inférieur de la rampe (6), ce qui va diriger à la manière d'une came la mâchoire inférieure vers l'avant, de sorte que les dents inférieures (14) vont se situer en face des dents supérieures (12) et qu'il existe un espace entre les dents supérieures et les dents inférieures et entre les lèvres, de sorte qu'en comparaison de la position normale, un espacement accru est créé entre la langue et le palais et la luette ainsi qu'entre la langue et la paroi arrière du pharynx, ce qui diminue la vitesse de l'air passant le long de la voie aérienne en empruntant cette ouverture (8) pendant la respiration, et les vibrations du palais et de la luette sont diminuées ou éliminées parce que les tissus mous, précédemment entraînés par le passage de l'air, ne vibrent plus ou n'oscillent plus ou ne vibrent qu'à une amplitude réduite.

2. Dispositif selon la revendication 1, dans lequel l'ouverture (8) s'étend à travers le dispositif en une position située au centre du dispositif.

3. Dispositif selon la revendication 1, dans lequel la rampe (6) forme un angle d'environ 60° quand on la regarde en élévation latérale.

4. Dispositif selon l'une quelconque des revendications précédentes, dans le cas duquel ce dispositif est moulé en une matière plastique synthétique du groupe des acryliques.

5. Dispositif selon la revendication 4, dans le cas duquel ce dispositif est moulé en du méthacrylate de méthyle.
